(19) Europäisches Patentamt
European Patent Office
Office européen des brevets

(11) Publication number: **0 359 196 A2**

(12) **EUROPEAN PATENT APPLICATION**

(21) Application number: **89116856.9**

(51) Int. Cl.[5]: **A61K 7/48**

(22) Date of filing: **11.09.89**

(30) Priority: **13.09.88 IT 8258588**

(43) Date of publication of application: **21.03.90 Bulletin 90/12**

(84) Designated Contracting States: **AT CH DE ES FR GB IT LI NL**

(71) Applicant: **Kawamura, Mariko**
**Via Montegrappa 4**
**I-31020 Villorba-Frazione Lancenigo(IT)**

(72) Inventor: **Kawamura, Mariko**
**Via Montegrappa 4**
**I-31020 Villorba-Frazione Lancenigo(IT)**

(74) Representative: **Modiano, Guido et al**
**MODIANO, JOSIF, PISANTY & STAUB**
**Modiano & Associati Via Meravigli, 16**
**I-20123 Milano(IT)**

(54) **Cosmetic formulation for skin care.**

(57) A cosmetic formulation for skin care which comprises, in addition to various emollient and moisturizing adjuvants or carriers conventionally used in cosmetic formulations, an aqueous or alcoholic extract of Luffa cylindrica, the extract being used in a proportion of 1 to 90% by weight with respect to the total weight of the formulation.

EP 0 359 196 A2

## COSMETIC FORMULATION FOR SKIN CARE

The present invention relates to a formulation for cosmetic use, in particular for skin care.

Numerous products are currently known, in the form of creams or lotions, suitable for caring for the skin and in particular against the forming of wrinkles or for decongesting the skin.

The aim of the present invention is to enrich the range of the cosmetic products which are already available for skin care by providing a new product which distinguishes itself by a particular effectiveness in treatment by virtue of its rapid and complete absorption into the skin.

Within the scope of the above-described aim, a further important object is to provide a cosmetic product of the above-mentioned kind which performs a moisturizing, decongesting and ultimately wrinkle-eliminating function, all of the above occurring with absolute lack of undesirable side effects, in particular allergic ones.

Treatment prolonged for a few months furthermore reduces the pigmentation of skin dyschromias, resulting from burns or wounds, sometimes up to a complete "restitutio ad integrum".

The above-mentioned aim and objects, as well as others which will become apparent hereinafter, are achieved by a cosmetic formulation for skin care, according to the invention, comprising at least one adjuvant and characterized in that it comprises from 1 to 90% by weight of an extract of Luffa cylindrica.

The Luffa extract which constitutes the active principle of the formulation according to the present invention may be an aqueous or alcoholic extract obtained for example by percolation, i.e. by slow filtering performed by forcing a solution to pass through thick layers of solid material.

The Luffa extract, depending on the extraction process as well as on further processing to concentrate or dilute the extract prior to use, may contain from 1 to 99% by weight of material extracted from Luffa cylindrica with the remainder being solvent used for extraction, water used to dilute said extract or mixtures thereof.

The adjuvants which are also comprised in the present formulation comprise emollient and moisturizing agents and inert carriers, chosen among those conventionally used in the field of cosmetic products.

The choice of said adjuvants depends to a large extent on the finished form of the formulation, which advantageously may be either a lotion or a fluid or solid cream.

In the case of a lotion, the moisturizing and emollient agents and the carrier are chosen among liquid substances.

In particular, the emollient and moisturizing agent is preferably chosen among organic acids and mono- or polyhydric alcohols which are well-accepted for cosmetic use, for example boric acid, citric acid, ethyl alcohol, glycerin and mixtures thereof.

Distilled water is preferably used as a carrier, though a mixture thereof with ethanol can be used.

When used in lotion form, the formulation comprises in general from 1 to 90% by weight of the Luffa cylindrica extract, preferably 30 to 40%.

Again for the lotion form, the formulation comprises in general 8 to 30% by weight of said adjuvant.

The remainder is a conventional additive such as for example benzoic acid ($C_6H_5COOH$) or another preservative commercially known by the trade-name Paraben or Paracombin which are present in an amount comprised between 0.1 and 10% by weight of the lotion, the remainder being distilled water.

If the formulation is in cream form, the adjuvant is preferably chosen among diglycerin dioleate, aluminum stearate, beeswax, white wax, spermaceti, whale wax, octyl dodecyl myristate, squalane, crystallized ozocerite, with the possible addition of glycerin, antioxidant and scent.

The formulation according to the invention may also comprise further conventional additives which are usually present in such cosmetic products, such as for example preservatives.

Such preservatives are used in an amount comprised between 0.1 to 5% by weight and can consist, for example, of products commercially known by the trade-names "Paraben" or "Paracombin".

If the formulation is in the form of milk or fluid cream, the adjuvant is preferably chosen among stearic acid, lanolin, 2-octyl dodecanol, sorbitan monostearate, polyoxyethylene sorbitan monostearate, triethanolamine, carboxylic polymers, propylene glycol, preservatives, and antioxidants, and is used in amounts varying from 9 to 70% by weight.

The active substance is again Luffa cylindrica, advantageously diluted in distilled water.

It has been observed that the formulation according to the invention performs an effective action for locally decongesting the skin, for reducing possible dyschromias and localized reddenings, re-equilibrating the correct moisture content of the skin, and in general eliminating and preventing the formation of wrinkles.

Even a prolonged use of the formulation by means of one or more localized applications per day is free from undesirable side effects such as allergic intolerances.

The following examples are given merely by way of illustration of possible cosmetic formulations based on the use of the extract of Luffa cylindrica and comprised within the scope of the present invention.

Considering a formulation with a weight of 1,000 g one has:

1) CITRIC ACID ($C_6H_8O_7$) 5-10 g
2) BORIC ACID ($H_3BO_3$) 10-20 g
3) ETHYL ALCOHOL ($C_2H_5OH$) 75-150 g
4) GLYCERIN ($C_3H_8O_3$) 30-100 g
5) ACTIVE SUBSTANCE (from percolation of Luffa cylindrica) 1-900 g
6) DISTILLED WATER 25-700 g
7) PRESERVATIVES: BENZOIC ACID ($C_6H_5COOH$) 1-5 g or PARABEN 1-4 g

If the formulation is in milk form, the following example is given, considering a total weight of 100 g:

1) STEARIC ACID ($C_{17}H_{35}COOH$) 1-10 g
2) LANOLIN 1-10 g
3)

2-OCTYL DODECANOL ( 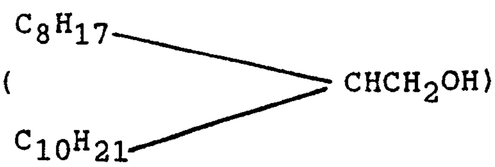 5-20 g

4) SORBITAN MONOSTEARATE (commercially known by the trade-name Span 60) 0.1-5 g
5) POLYOXYETHYLENE SORBITAN MONOSTEARATE (commercially known by the trade-name Tween 60) 1-3 g
6) TRIETHANOLAMINE ($(CH_2CH_2OH)_3N$) 0.1 - 2 g
7) CARBOXYLIC POLYMER (commercially known by the trade-name CARBOPOL) 0.1-0.5 g
8) PROPYLENE GLYCOL ($CH_3$ CHOH $CH_2OH$) 1-10 g
9) PRESERVATIVE (ethyl-p-hydroxybenzoate) (commercially known by the trade-name Paraben) 0.1-4 g
(Paracombin ® ) 0.1-0.8 g
10) ANTIOXIDANT
(vitamin E = tocopherol) 0.1-1 g
11) ACTIVE SUBSTANCE LUFFA CYLINDRICA IN DISTILLED WATER IN AN AMOUNT TO BRING TOTAL WEIGHT UP TO 100 g

If the formulation is in cream form, an example is given assuming a total weight of 100 g:

1) DIGLYCERIN DIOLEATE 1-10 g ($C_{17}H_{35}COOH$ CH $CH_2$ O $CH_2$ O $CH_2$ $CH_2$ O CH COOH $C_{17}$ $H_{35}$)
2) ALUMINUM STEARATE ($Al(C_{18}H_{35}O_2)_3$) 0.1-2 g
3) BEESWAX OR WHITE WAX 0.5-5 g
4) SPERMACETI (whale wax: Physter macrocephalus Linnè) 0.5-8 g
5)

2-OCTYL DODECYL MYRISTATE ($CH_3(CH_2)_{12}$ $COOCH_2$ 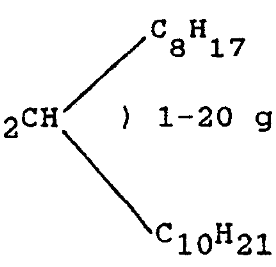 ) 1-20 g

6) SQUALANE ($C_{30}H_{62}$) 1- 15 g
7) CRYSTALLIZED OZOCERITE
(known by the trade-name CERESIN) 1-10 g
8) GLYCERIN ($C_3H_8O_3$) O-10 g
9) ANTIOXIDANT (VITAMIN E = TOCOPHEROL) 0.1 - 3 g
10) PRESERVATIVE 0.1-5 g
11) SCENT 0.1-5 g

12) ACTIVE SUBSTANCE LUFFA CYLINDRICA IN DISTILLED WATER IN AN AMOUNT TO BRING TOTAL WEIGHT UP TO 100 g

If the formulation is in liquid form, for use for example as an after-shave, an example is given assuming a total weight of 1000 g:

1) Citric acid ($C_6H_8O_7$) 5-10 g
2) Boric acid ($H_3BO_3$) 10-30 g
3) Ethyl alcohol ($C_2H_5OH$) 200-700 g
4) Glycerin ($C_3H_8O_3$) 20-100 g
5) Active substance (from percolation of Luffa cylindrica) 50-400 g
6) Distilled water 10-500 g
7) Preservative (ethyl-p-hydroxybenzoate) 1-2 g (known commercially by the trade-name Paraben)
8) Alcloxa ® (Aluminum chloro hydroxy allantoate) 1-2 g
9) scent in an amount to bring total weight up to 1000 g.

Advantageously, the extract of Luffa cylindrica is an aqueous extract obtained by boiling leaves and/or roots and/or percolate from the trunk of Luffa cylindrica in water and by filtering the mixture and cooling the separated aqueous liquid extract.

Likewise, the extract of Luffa cylindrica can be an alcoholic or aqueous alcoholic extract.

Depending on any further concentration or dilution, such extract contains between 1 and 99% by weight of material extracted from Luf fa cylindrica with the remainder being solvent used for extraction or any water added or mixtures thereof.

## Claims

1. Cosmetic formulation for skin care, characterized in that it comprises least one adjuvant and from 1 to 90% by weight of an extract of Luffa cylindrica.

2. Formulation according to claim 1, characterized in that said extract of Luffa cylindrica comprises between 1 and 99% by weight of material extracted from Luffa cylindrica with the remainder being solvent used for extraction, water used to dilute said extract or mixtures thereof.

3. Formulation according to claim 1 or 2, characterized in that said extract of Luffa cylindrica is obtained by percolation.

4. Formulation according to claims 1 and 3, characterized in that it comprises said extract of Luffa cylindrica in the amount of 30 to 40% by weight.

5. Formulation according to claims 1, 2, 3 or 4, characterized in that it comprises at least one of said adjuvants selected from emollient and moisturizing agents and carriers for cosmetic use.

6. Formulation according to claims 1 and 4, characterized in that it is in lotion form and comprises at least one of said adjuvants selected from emollient and moisturizing agents and carriers for cosmetic use, said moisturizing and emollient agents being selected from organic acids and mono- and poly-hydric alcohols.

7. Formulation according to claim 6, characterized in that it comprises at least one of said moisturizing and emollient agents selected from citric acid, boric acid, ethyl alcohol, glycerin and mixtures thereof and is used in an amount between 8 and 30% by weight of said formulation.

8. Formulation according to one or more of the preceding claims, characterized in that it is in cream form and comprises at least one of said adjuvants selected from diglycerin dioleate, aluminum stearate, beeswax, white wax, whale wax, 2-octyl dodecyl myristate, squalane, an adjuvant commercially known by the trade-name Ceresin and mixtures thereof.

9. Formulation according to one or more of the preceding claims, characterized in that said extract of Luffa cylindrica is an aqueous extract obtained by boiling leaves and/or roots and/or percolate from the trunk of Lu fa cylindrica in water and by filtering the mixture and cooling the separated aqueous liquid extract.

10. Formulation according to one or more of the preceding claims, characterized in that said extract of Luffa cylindrica is an alcoholic extract obtained by boiling leaves and/or roots and/or percolate from the trunk of Luffa cylindrica in alcohol or aqueous alcoholic solution and by filtering the mixture and cooling the separated aqueous liquid extract.

11. Formulation according to one or more of the preceding claims, characterized in that it furthermore comprises at least one preservative in an amount between 0.1 and 1% by weight of said formulation.

12. Formulation according to claims 1 and 10, characterized in that said preservative is selected from benzoic acid and salicylic acid.

13. Formulation according to one or more of the preceding claims, characterized in that it is in the form of a solution, for after-shave, and that it comprises said extract of Luffa cylindrica in an amount between 1 and 75% by weight of said formulation and said at least one adjuvant in an amount between 23.5 and 80% by weight of said formulation, said solution comprising aluminum chloro hydroxy allantoate.

14. Formulation according to one or more of the preceding claims, characterized in that it furthermore comprises at least one antioxidant.

15. Formulation according to one or more of the preceding claims, characterized in that it furthermore comprises scent.

16. Formulation according to one or more of the preceding claims, characterized in that the composition by weight per cent is 0.5-1% citric acid, 1-2% boric acid, 7.5-15% ethyl alcohol, 3-10% glycerin, 0.1-90% extract from Luffa cylindrica, 2.5-70% water and 0.1-0.5% preservatives.

17. Formulation according to one or more of the preceding claims, characterized in that the composition by weight per cent is 1-10% stearic acid, 1-10% lanolin, 5-20-% 2-octyl dodecanol, 0.1-5% sorbitan monostearate, 1-3% polyoxyethylene sorbitan monostearate, 0.1-2% triethanolamine, 0.1-0.5% carboxylic polymer, 1-10% propylene glycol, 0.1-4% preservative, 0.1-1% antioxidant and extract of Luffa cylindrica in water in an amount to bring total percentage up to 100%.

18. Formulation according to one or more of the preceding claims, characterized in that the composition by weight per cent is 1-10% diglycerin dioleate, 0.1-2% aluminum stearate, 0.5-5% beeswax or white wax, 0.5-8% spermaceti, 1-20% 2-octyl dodecyl myristate, 1-15% squalane, 1-10% crystallized ozocerite, O-10% glycerin, 0.1-3% antioxidant, 0.1-5% preservative, 0.1-5% scent and extract of Luffa cylindrica in water in an amount to bring the total percentage up to 100%.

19. Formulation according to one or more of the preceding claims, characterized in that the composition by weight per cent is 0.5-1% citric acid, 1-3% boric acid, 2070% ethyl alcohol, 2-10% glycerin, 5-40% extract of Luffa cylindrica, 1-50% water, 0.1-0.2% preservative, 0.1-0.2% aluminum chloro hydroxy allantoate and scent in an amount to bring total percentage up to 100%.